(19) Europäisches Patentamt
European Patent Office
Office européen des brevets

(11) Veröffentlichungsnummer : **0 136 658**
**B1**

(12) # EUROPÄISCHE PATENTSCHRIFT

(45) Veröffentlichungstag der Patentschrift : **26.04.89**

(51) Int. Cl.⁴ : **C 07 D207/26**, C 07 D403/06, A 61 K 31/40

(21) Anmeldenummer : **84111512.4**

(22) Anmeldetag : **27.09.84**

(54) **1-Benzyl-aminoalkyl-pyrrolidinone und ihre Säureadditionssalze, Verfahren zu ihrer Herstellung und Arzneimittel.**

(30) Priorität : **04.10.83 DE 3336024**

(43) Veröffentlichungstag der Anmeldung : **10.04.85 Patentblatt 85/15**

(45) Bekanntmachung des Hinweises auf die Patenterteilung : **26.04.89 Patentblatt 89/17**

(84) Benannte Vertragsstaaten : **AT BE CH DE FR GB IT LI LU NL SE**

(56) Entgegenhaltungen :
**DE-A- 2 923 975**
**FR-A- 2 387 218**
**JP-A-78 046 966**
**US-A- 3 459 738**
**US-A- 4 369 139**
**CHEMICAL ABSTRACTS, Band 89, Nr. 13, 25. September 1978, p. 879, no. 109078v, COLUMBUS, OHIO (US)**
**CHEMICAL ABSTRACTS, Band 89, Nr. 13, 25. September 1978, p. 880, no. 109080q, COLUMBUS, OHIO (US)**
**Die Akte enthält technische Angaben, die nach dem Eingang der Anmeldung eingereicht wurden und die nicht in dieser Patentschrift enthalten sind.**

(73) Patentinhaber : **BOEHRINGER INGELHEIM KG**
**Postfach 200**
**D-6507 Ingelheim am Rhein (DE)**
**BE CH DE FR IT LI LU NL SE AT**
**BOEHRINGER INGELHEIM INTERNATIONAL G.M.B.H.**
**Postfach 20**
**D-6507 Ingelheim am Rhein (DE)**
**GB**

(72) Erfinder : **Weber, Karl-Heinz, Dr.**
**Kaiser-Karl-Strasse 11**
**D-6535 Gau-Algesheim (DE)**
Erfinder : **Walther, Gerhard, Dr.**
**Pfarrer-Heberer-Strasse 37**
**D-653 Bingen/Rhein (DE)**
Erfinder : **Schneider, Claus, Dr.**
**Albrecht-Dürer-Strasse 19**
**D-6507 Ingelheim am Rhein (DE)**
Erfinder : **Hinzen, Dieter, Prof. Dr.**
**Stromberger Strasse 36**
**D-653 Bingen/Rhein (DE)**
Erfinder : **Kuhn, Franz Josef, Dr.**
**Beethovenstrasse 11**
**D-6535 Gau-Algesheim (DE)**
Erfinder : **Lehr, Erich, Dr.**
**In der Toffel 5**
**D-6531 Waldalgesheim (DE)**

**EP 0 136 658 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 1-Benzyl-aminoalkyl-pyrrolidinone und deren Säureadditionssalze, Verfahren zu ihrer Herstellung und Arzneimittel, die diese Verbindungen als Wirkstoff enthalten.

Die neuen Verbindungen vermögen der Einschränkung des Kurzzeitgedächtnisses nach Gabe von muscarinen cholinergen Antagonisten entgegenzuwirken und besitzen darüber hinaus eine ausgeprägte Antihypoxie-Aktivität.

Als strukturell ähnlich gebaute Nootropica sind das 1-Carbamoylmethyl-pyrrolidin-2-on (Piracetam), das 1-(p-Methoxybenzoyl)-pyrrolidin-2-on (Aniracetam) und das 1-Carbamoylmethyl-4-hydroxy-pyrrolidin-2-on (Oxiracetam) bereits in der Literatur beschrieben, siehe B.J.R. Nicolaus, Drug Development Res. 2, 464 (1982), P.L. Paytasch, J. Amer, Chem. Soc. 72, 1415 (1950).

Es wurde nun gefunden, daß die Einführung einer Aminomethylgruppe in das Pyrrolidinonmolekül eine gegenüber den bekannten Stoffen wesentliche Verbesserung der Wirkung mit sich bringt.

Gegenstand der Erfindung sind neue 1-Benzyl-aminoalkyl-pyrrolidinone der allgemeinen Formel

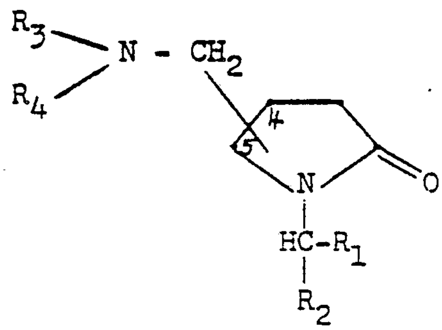

(I)

worin

$R_1$ Wasserstoff oder Methyl,

$R_2$ einen Phenylrest, der ein- oder zweifach durch Alkoxy, Fluor, Chlor, Brom, Trifluormethyl, Alkyl, Hydroxy oder Nitro substituiert sein kann oder einen Pyridylrest und

$R_3$ und $R_4$, die gleich oder verschieden sein können, Wasserstoff oder einen Alkylrest mit 1 bis 2 Kohlenstoffatomen bedeuten oder beide Reste $R_3$ und $R_4$ zusammen mit dem Stickstoffatom einen gesättigten 5- oder 6-Ring, der als weiteres Heteroatom ein O- oder N-Atom enthalten und gegebenenfalls durch Methyl substituiert sein kann oder einen Imidazolring bilden, und wobei die Aminoalkylgruppe in 4- oder 5-Stellung steht und deren Säureadditionssalze.

Aus der japanischen Patentanmeldung JP-A-46966 ist 5-(Aminomethyl)-1-(α-methylbenzyl)-pyrrolidin-2-on als Zwischenverbindung zur Herstellung von pharmazeutisch wirksamen Verbindungen bekannt, wobei die pharmakologische Wirksamkeit dieser Verbindung selbst nicht offenbart ist.

In der allgemeinen Formel I steht die Bezeichnung « Alkyl » für eine geradkettige oder verzweigte Alkylgruppe mit 1-4 Kohlenstoffatomen und die Bezeichnung « Alkoxy » für einen Rest mit 1-2 Kohlenstoffatomen ; der als Bedeutung für $R_2$ genannte Pyridylring kann in 2-, 3- oder 4-Stellung mit der Methylenbrücke verknüpft sein.

Die Endprodukte der allgemeinen Formel I können gewünschtenfalls nach üblichen Methoden in ihre physiologisch unbedenklichen Säureadditionssalze überführt werden.

Als Säuren eignen sich hierfür sowohl anorganische Säuren wie Halogenwasserstoffsäuren, Schwefelsäure, Phosphorsäure und Aminosulfonsäure, als auch organische Säuren wie Ameisensäure, Essigsäure, Propionsäure, Milchsäure, Glykolsäure, Glukonsäure, Maleinsäure, Fumarsäure, Bernsteinsäure, Weinsäure, Benzoesäure, Salicylsäure, Zitronensäure, Ascorbinsäure, p-Toluolsulfonsäure oder Oxyäthansulfonsäure.

Bevorzugte Verbindungen der allgemeinen Formel I sind solche, bei denen $R_1$ Wasserstoff, $R_2$ einen gegebenenfalls durch Fluor, Chlor, Methyl oder Methoxy in o- oder p-Stellung substituierten Phenylrest und $R_3$ und $R_4$ Wasserstoff oder Methyl bedeuten.

Gegenstand der Erfindung sind ferner Verfahren zur Herstellung der Verbindungen der allgemeinen Formel I. Verbindungen, in denen die Reste $R_3$ und/oder $R_4$ von Wasserstoff verschieden sind, erhält man beispielsweise ausgehend von Hydroxymethyl-Pyrrolidinonen der allgemeinen Formel

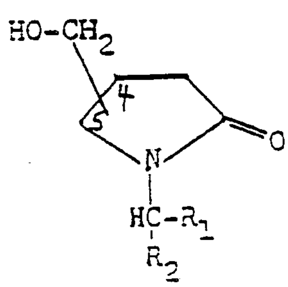

(II)

worin $R_1$ und $R_2$ die oben angegebene Bedeutung haben, durch Umsetzung mit einem Thionyl- oder einem Phosphor-Halogenid oder einem Tosyl- oder Mesylhalogenid zu einer Verbindung der allgemeinen Formel III

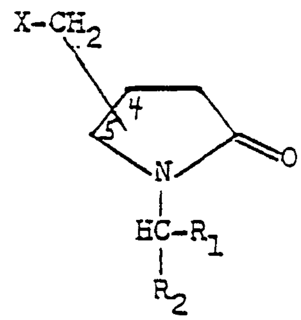

(III)

worin $R_1$ und $R_2$ die oben angegebene Bedeutung haben und X ein Halogenatom oder den Tosyloxybeziehungsweise Mesyloxyrest bedeutet und anschließender Reaktion mit einem primären oder sekundären Amin der allgemeinen Formel

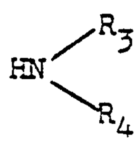

(IV)

worin $R_3$ und $R_4$ die oben angegebene Bedeutung haben.

Hierbei wird eine Verbindung der allgemeinen Formel II entweder mit einem Thionyl- oder Phosphor-Halogenid in die entsprechende 4-Halogenmethylverbindung beziehungsweise mit einem Tosyl- oder Mesyl-Halogenid in den entsprechenden 4-Tosyl- beziehungsweise 4-Mesylester überführt. Die Reaktion findet vorzugsweise unter Verwendung eines inerten organischen Lösungsmittels, wie zum Beispiel Chloroform, Methylenchlorid, Tetrahydrofuran oder Dimethylformamid bei Temperaturen zwischen Raumtemperatur und dem Siedepunkt des verwendeten Lösungsmittels statt. Im Falle der Veresterung wird zweckmäßigerweise eine tertiäre organische Base, zum Beispiel Triäthylamin oder Pyridin, zugesetzt. Die als Zwischenprodukte entstehenden 4-Halogenmethylverbindungen beziehungsweise die 4-Tosyl- oder 4-Mesylester können isoliert oder aber in situ weiter umgesetzt werden. Man erhält bei ihrer Behandlung mit primären oder sekundären Aminen die entsprechenden Endprodukte der allgemeinen Formel I. Die Reaktion kann in Tetrahydrofuran, Dioxan, Acetonitril oder vorzugsweise in Dimethylformamid durchgeführt werden ; die Temperaturen liegen etwa zwischen 50 und 150 °C, wobei die einzelnen Reaktionsbedingungen von der Basizität und dem Siedepunkt des Amins abhängen ; die Umsetzung kann auch ohne Verwendung eines Lösungsmittels in einem Überschuß des Amins durchgeführt werden. Bei niedrig siedenden Aminen muß die Reaktion unter Umständen im Autoklaven erfolgen.

Die Synthese von Endprodukten der allgemeinen Formel I mit freier Aminogruppe erfolgt vorzugsweise durch Umsetzung einer Verbindung der allgemeinen Formel III mit Phthalimid beziehungsweise einem Alkalisalz des Phthalimids und Spaltung der so erhaltenen 4-Phthalimidomethylverbindung mit Hydrazin.

Die Umsetzung mit Phthalimid findet in einem inerten organischen Lösungsmittel wie Acetonitril oder einem Alkohol oder bevorzugt in Dimethylformamid bei erhöhter Temperatur — bis zum Siedepunkt des Reaktionsgemisches — statt. Die 4-Phthalimidomethylverbindung kann isoliert oder in situ weiter umgesetzt werden ; die Abspaltung der Phthalimidgruppe kann mit Hydrazin erfolgen. Bei der Abspaltung können Alkohole, Tetrahydrofuran, Dioxan oder Dimethylformamid oder Mischungen aus Alkoholen und Dimethylformamid als Lösungsmittel dienen. Oft läuft die Reaktion bereits bei Raumtemperatur ab ; gegebenenfalls wird eine höhere Temperatur gewählt, um die Reaktion zu beschleunigen.

Eine weitere Herstellungsmöglichkeit für die Norverbindung besteht in der Hydrierung einer Verbindung der allgemeinen Formel V.

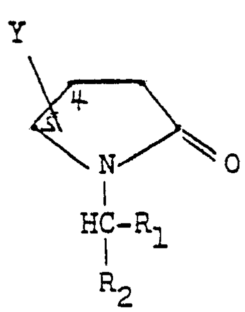

(V)

worin

$R_1$ und $R_2$ die oben angegebene Bedeutung haben und

Y einen der Reste $—CH_2N_3$ oder —CN bedeutet.

Die Hydrierung erfolgt vorzugsweise mittels Raney-Nickel in Methanol oder auch mit Palladium in Methanol/Wasser, im Fall der Hydrierung des Nitrils gegebenenfalls unter Ammoniakzugabe.

Ein Azid der allgemeinen Formel V ($Y = —CH_2N_3$) erhält man durch Umsetzung einer Verbindung der allgemeinen Formel III, worin X ein Halogenatom oder die Tosylbeziehungsweise Mesylgruppe bedeutet, mit Natriumazid in einem inerten organischen Lösungsmittel, beispielsweise einem Alkohol oder einem Äther wie Tetrahydrofuran oder Dioxan.

Ein Nitril der allgemeinen Formel V (Y = —CN) wird zum Beispiel durch Umsetzung einer in Acetonitril gelösten Säure der allgemeinen Formel

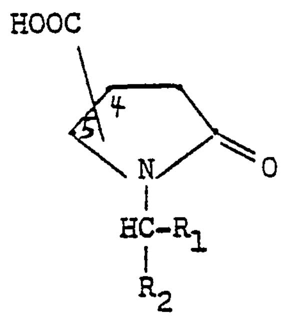

(VI)

worin $R_1$ und $R_2$ die oben genannte Bedeutung haben mit Chlorsulfonylisocyanat und anschließend mit Triäthylamin oder durch Dehydratisierung des entsprechenden Carbonsäureamids mit z. B. $POCl_3$ hergestellt (vgl. H. Vorbrüggen, Tetrahedron Letters 1968, S. 1631-1634).

Die neuen Verbindungen der allgemeinen Formel I besitzen ein Asymmetriezentrum und liegen daher als Racemate vor. Diese Racemate können in üblicher Weise, z. B. durch Salzbildung mit optisch aktiven Säuren, in die entsprechenden Diastereomeren überführt werden, die dann in die optisch aktiven Endprodukte umgewandelt werden können.

Gegebenenfalls können die Alkohole der allgemeinen Formel II durch Esterbildung mit optisch aktiven Säuren in die entsprechenden Diastereomerengemische überführt werden, die sich dann auf übliche Weise, z. B. durch Säulenchromatographie oder fraktionierte Kristallisation, in die entsprechenden Diastereomeren auftrennen lassen. Nach Verseifung dieser Ester erhält man die enantiomeren Alkohole, aus denen nach einem der Verfahren a) oder b) die entsprechenden Amine hergestellt werden.

Ausgehend von den reinen enantiomeren Nor-Verbindungen können durch reduktive Alkylierung, z. B. mit Acetaldehyd/$H_2$/Katalysator die entsprechenden optisch reinen Diäthylverbindungen oder mit Formaldehyd/Ameisensäure die entsprechenden optisch reinen Dimethylverbindungen erhalten werden.

Die Ausgangsstoffe für die vorstehend geschilderten Verfahren sind bekannt oder können nach bekannten Verfahren erhalten werden.

So wird die Pyrrolidinoncarbonsäure der allgemeinen Formel VI durch Umsetzung äquimolarer Mengen Itaconsäure und eines entsprechenden Amins hergestellt. Aus der Säure kann über den Ester durch selektive Reduktion mit einem komplexen Alkaliborhydrid die Ausgangsverbindung II erhalten werden (vgl. Deutsche Patentanmeldung P-33 26724.3).

Im Falle der 5-Aminoalkylverbindungen kann man von den käuflichen 5-Pyrrolidinocarbonsäuren ausgehen, diese verestern, am Stickstoff mit gegebenenfalls substituiertem Benzylhalogenid alkylieren und, wie oben beschrieben, weiter umsetzen.

Nach den geschilderten Verfahren können beispielsweise die folgenden Endprodukte, gegebenenfalls in Form ihrer Säureadditionssalze und gegebenenfalls in Form ihrer reinen Enantiomeren, erhalten werden :

1-Benzyl-4-aminomethyl-pyrrolidin-2-on
1-(4-Methoxybenzyl)-4-aminomethyl-pyrrolidin-2-on
1-(3,4-Dimethoxybenzyl)-4-aminomethyl-pyrrolidin-2-on
1-(4-Methylbenzyl)-4-aminomethyl-pyrrolidin-2-on
1-(4-Fluorbenzyl)-4-aminomethyl-pyrrolidin-2-on
1-(4-Chlorbenzyl)-4-aminomethyl-pyrrolidin-2-on
1-(3-Trifluormethylbenzyl)-4-aminomethyl-pyrrolidin-2-on
1-(4-Pyridylmethyl)-4-aminomethyl-pyrrolidin-2-on
1-(α-Methylbenzyl)-4-aminomethyl-pyrrolidin-2-on
1-Benzyl-4-piperidinomethyl-pyrrolidin-2-on
1-(4-Fluorbenzyl)-4-morpholino-pyrrolidin-2-on
1-Benzyl-4-(N-methylpiperazino)-pyrrolidin-2-on
1-Benzyl-4-imidazol-1-yl-pyrrolidin-2-on

1-Benzyl-4-methylaminomethyl-pyrrolidin-2-on
1-(p-Fluorbenzyl)-4-dimethylaminomethyl-pyrrolidin-2-on
1-Benzyl-4-diäthylaminomethyl-pyrrolidin-2-on
1-(4-Nitrobenzyl)-4-aminomethyl-pyrrolidin-2-on
1-(4-Hydroxybenzyl)-4-aminomethyl-pyrrolidin-2-on
1-(o-Chlorbenzyl)-4-aminomethyl-pyrrolidin-2-on
1-(o-Chlorbenzyl)-4-diäthylaminomethyl-pyrrolidin-2-on
1-(p-Methylbenzyl)-4-diäthylaminomethyl-pyrrolidin-2-on
1-Benzyl-5-dimethylaminomethyl-pyrrolidin-2-on
1-Benzyl-5-diäthylaminomethyl-pyrrolidin-2-on
1-Benzyl-5-morpholinomethyl-pyrrolidin-2-on
1-Benzyl-5-(4-methylpiperazino)-methyl-pyrrolidin-2-on
1-Benzyl-5-pyrrolidinomethyl-pyrrolidin-2-on
1-(4-Methylbenzyl)-5-dimethylaminomethyl-pyrrolidin-2-on
1-(4-Methylbenzyl)-5-diäthylaminomethyl-pyrrolidin-2-on
1-(p-Chlorbenzyl)-5-dimethylaminomethyl-pyrrolidin-2-on
1-(p-Chlorbenzyl)-5-diäthylaminomethyl-pyrrolidin-2-on
1-(3,4-Dichlorbenzyl)-5-dimethylaminomethyl-pyrrolidin-2-on
1-(3,4-Dichlorbenzyl)-5-diäthylaminomethyl-pyrrolidin-2-on
1-(p-Methoxybenzyl)-5-dimethylaminomethyl-pyrrolidin-2-on
1-(p-Methoxybenzyl)-5-diäthylaminomethyl-pyrrolidin-2-on
1-Benzyl-5-aminomethyl-pyrrolidin-2-on

Die neuen Pyrrolidinon-Derivate wurden in Tierexperimenten bezüglich ihrer Wirksamkeit untersucht, Zustandsformen eingeschränkter cerebraler Leistungsfähigkeit aufzuheben beziehungsweise zu mindern.

Die Verbindungen weisen in orientierenden Verträglichkeitsuntersuchungen an der Maus in Dosierungen bis zu 2 g/kg (einmalige orale Applikation) keine akute Toxizität (14 Tage Nachbeobachtung) auf. Sie zeigen tierexperimentell ausgezeichnete Wirkungen auf spontane kognitive Leistungen, wie experimentell eingeschränkte Lern- und Gedächtnisvorgänge. In Versuchen mit Einschränkung des Kurzzeitgedächtnisses beziehungsweise Behinderung des Übergangs von Inhalten des Kurzzeit- in das Langzeitgedächtnis durch Gabe eines muscarinen cholinergen Antagonisten (Scopolamin 0,6 mg/kg i.p. ; siehe auch Psychopharmacology 78, 104 - 111 (1982)), sind die Verbindungen in der Lage, dieser pharmakologisch induzierten cerebralen Insuffizienz entgegenzuwirken, beziehungsweise sie aufzuheben.

Die Lernfähigkeit von Ratten in einer aktiven Vermeidedressur (J. pharmacol. Methods, 8, 255 - 263 (1983)) wird ebenso verbessert wie ihre spontane Habituation beziehungsweise explorierende Orientierungsaktivität in einer neuen Umgebung.

Bei der Prüfung der Überlebensfähigkeit von Tieren in einer geschlossenen Kammer (Hypoxie-Toleranztest), welche mit einem Gasgemisch bestehend aus 96,5 % $N_2$ und 3,5 % $O_2$ durchströmt wurde, wiesen die mit den neuen Substanzen vorbehandelten Tiere eine statistisch hochsignifikant größere Überlebensfähigkeit auf als Kontrolltiere beziehungsweise mit Piracetam vorbehandelte Tiere. Darüber hinaus war die mit dieser Methode geprüfte hirnprotektive Wirkung der Substanzen bereits in einer Dosis von 50 mg/kg p.o. ausgeprägt.

Die neuen Pyrrolidinon-Derivate wurden in ihrer Wirksamkeit mit andersstrukturierten Pyrrolidinonen verglichen, welche im Rahmen der cerebralen Insuffizienz beziehungsweise des hirnorganischen Psychodroms, der posttraumatischen und alkoholischen Hirnschädigung usw., in der Humanmedizin bereits als Arzneimittel Anwendung finden (Piracetam) beziehungsweise zur Zeit klinisch erprobt werden (Aniracetam).

Die neuen Verbindungen sind sowohl bezüglich der wirksamen Dosis als auch der im Tierexperiment erzielten Leistungsverbesserung den genannten Substanzen deutlich überlegen.

Die neuen Verbindungen können allein oder in Kombination mit anderen erfindungsgemäßen Wirskstoffen, gegebenenfalls auch in Kombination mit weiteren pharmakologisch aktiven Wirkstoffen, z.B. weiteren Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen

kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z. B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z. B. unter Zusatz von Konservierungsmitteln, wie p-Hydroxybenzoaten, oder Stabilisatoren, wie Alkalisalzen der Äthylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die nachfolgenden Beispiele illustrieren die vorliegende Erfindung, ohne sie jedoch in ihrem Umfang zu beschränken :

## Beispiel 1

1-Benzyl-4-aminomethyl-pyrrolidin-2-on

54 g (0,16 Mol) 4-Phthalimidomethyl-1-benzyl-pyrrolidin-2-on werden in 1,3 l Äthylalkohol nach Zusatz von 32 g Hydrazinhydrat 4 Stunden bei Raumtemperatur gerührt. Der Niederschlag (Phthalsäurehydrazid) wird abgesaugt und das Filtrat eingedampft. Man setzt 500 ml Methylenchlorid zu dem Rückstand und schüttelt dreimal mit 100 ml Wasser aus. Die organische Phase wird getrocknet und eingedampft. Den verbleibenden Rückstand löst man in 500 ml Methanol und fügt bei Siedehitze unter Rühren 20 g (0,17 Mol) feste Fumarsäure portionsweise hinzu. Beim Abkühlen scheiden sich farblose Kristalle ab, die man absaugt und mit Methanol und Äther nachwäscht. Ausbeute : 20-25 g (48-60 % d.Th.) vom Fp. 209-211 °C. Die Verbindung enthält 1/2 Mol Fumarsäure.

Das Ausgangsmaterial wird wie folgt erhalten :

a) 94 g (0,46 Mol) 1-Benzyl-4-hydroxymethyl-pyrrolidin-2-on werden mit 700 ml Methylenchlorid und 40 ml (0,54 Mol) Thionylchlorid 25 Stunden unter Rückflußkochen gerührt und das Reaktionsgemisch anschließend unter Kühlung mit verdünntem Ammoniak neutralisiert. Nach Abtrennen, Trocknen und Eindampfen hinterbleiben 85-90 g eines dunkeln Öls, welches direkt zur weiteren Umsetzung verwendet wird.

b) 43,5 g (0,195 Mol) rohes 1-Benzyl-4-chlormethyl-pyrrolidin-2-on, 36 g (0,195 Mol) Phthalimidkalium und 700 ml Dimethylformamid werden 2 Stunden unter Rückfluß gekocht. Das Reaktionsgemisch wird anschließend im Vakuum eingedampft und der Rückstand in Methylenchlorid aufgenommen. Man schüttelt mehrmals mit Wasser aus, trocknet die organische Phase und erhält nach Chromatographie über $SiO_2$ 45 g (70 % d.Th.) der Phthalimidoverbindung vom Fp. 108-109 °C.

## Beispiel 2

1-Benzyl-4-aminomethyl-pyrrolidin-2-on

a) 58 g (0,29 Mol) 1-Benzyl-4-nitrilo-pyrrolidin-2-on werden in Methanol gelöst und unter Zusatz von flüssigem Ammoniak über Raney-Nickel katalytisch hydriert. Nach dem Einengen der Reaktionslösung wird in Methanol gelöst, von Katalysatorresten abfiltriert und nach dem Erwärmen auf ca. 50 °C mit 17 g Fumarsäure versetzt. Unter Rühren geht die Fumarsäure kurzzeitig in Lösung, danach beginnt die Kristallisation des 1-Benzyl-4-aminomethyl-pyrrolidin-2-on-fumarats. Ausbeute : 68 g (= 91 % d.Th.) ; Fp. 192-194 °C.

b) Die Nitriloverbindung wird in 96 % Ausbeute aus dem entsprechenden Amid vom Fp. 162-166 °C durch Dehydratisierung mittels $POCl_3$ in Dimethylformamid bei ca. 60 °C als Öl erhalten.

## Beispiel 3

Racematspaltung von 1-Benzyl-4-aminomethyl-pyrrolidin-2-on

a) 24,0 g (0,117 Mol) 1-Benzyl-4-aminomethyl-pyrrolidin-2-on werden in 200 ml heißem Methanol gelöst, 17,6 g (0,117 Mol) L(+)-Weinsäure werden ebenfalls in 200 ml heißem Methanol gelöst. Die

beiden Lösungen werden zusammengegeben und unter Rühren auf Raumtemperatur abkühlen lassen, wobei das Salz auskristallisiert. Die Kristalle werden kalt abgesaugt, mit kaltem Methanol nachgewaschen und getrocknet. Ausbeute : 18,0 g 4-Aminomethyl-1-benzyl-pyrrolidin-2-on-tartrat, Fp. 204-206 °C (aus Methanol), $\alpha_D$ = + 6,3° (c = 1,0 ; Wasser).

b) Zur Umwandlung des Tartrats in die Base wird das Tartrat kalt in 20 ml Wasser und 10 ml konzentrierter Natronlauge gelöst, dreimal mit Methylenchlorid extrahiert, die vereinigten Methylenchloridphasen über $MgSO_4$ getrocknet und das Lösungsmittel i.V. abgezogen. Man erhält das (—)-4-Aminomethyl-1-benzyl-pyrrolidin-2-on, $\alpha_D$ = — 8,4° (c = 1,0 ; Wasser).

c) Die bei der unter a) angegebenen Aufarbeitung anfallenden Mutterlaugen werden i.V. eingeengt. Man erhält 38,0 g des Tartrats, das kalt in 140 ml Wasser und 50 ml konzentrierter Natronlauge aufgenommen und dreimal mit Methylenchlorid ausgeschüttelt wird. Die vereinigten Methylenchloridphasen werden über $MgSO_4$ getrocknet und das Lösungsmittel i.V. abgezogen. Man erhält : 19,3 g Base, die, wie unter a) beschrieben, mit D-(—)-Weinsäure in das entsprechende Tartrat überführt wird. Ausbeute : 19,0 g vom Fp. 204-205 °C.

d) Die Umwandlung des Tartrats in die Base erfolgt wie unter b) beschrieben. Man erhält 5,7 g (+)-4-Aminomethyl-1-benzyl-pyrrolidin-2-on mit einem Drehwert $\alpha_D$ = + 8,4° (c = 1,0 ; Wasser).

## Beispiel 4

(—)-1-Benzyl-4-dimethylaminomethyl-pyrrolidin-2-on

4,0 g (0,02 Mol) (—)-1-Benzyl-4-aminomethyl-pyrrolidin-2-on und 5,4 g 85 %ige Ameisensäure werden mit 4,8 ml Formalinlösung versetzt und über Nacht bei 100 °C (Ölbad) gerührt. Danach wird die überschüssige Säure i.V. abdestilliert, der Rückstand in Wasser aufgenommen, mit konzentrierter Natronlauge alkalisch gestellt und dreimal mit Methylenchlorid ausgeschüttelt. Die vereinigten Methylenchloridphasen werden mit Wasser gewaschen, über Natriumsulfat getrocknet, das Lösungsmittel i.V. eingeengt und der Rückstand über eine $SiO_2$-Säule filtriert (Fließmittel : Methylenchlorid : Methanol = 97 :3). Die einheitliche Fraktion wird i.V. eingeengt. Man erhält die Titelverbindung in einer Ausbeute von 3,5 g (als Öl).

$\alpha_D$ = — 7,6° (c = 1,0 ; Methanol)

$\alpha_D$ = — 16,8° (c = 1,0 ; Wasser).

Analog Beispiel 4 erhält man aus 5,8 g (0,028 Mol) (+)-1-Benzyl-4-aminomethyl-pyrrolidin-2-on, 7,9 g 85 %iger Ameisensäure und 7 ml Formalinlösung 6,1 g (+)-1-Benzyl-4-dimethylaminomethyl-pyrrolidin-2-on, $\alpha_D$ = + 7,9° (c = 1,0 ; Methanol).

## Beispiel 5

1-Benzyl-4-di-äthylaminomethyl-pyrrolidin-2-on

14 g (0,06 Mol) rohes 1-Benzyl-4-chlormethyl-pyrrolidin-2-on, hergestellt nach Beispiel 1a), 10 g Diäthylamin und 50 ml Dimethylformamid werden 2 Stunden bei 150 °C im Autoklaven gerührt oder geschüttelt. Man dampft im Vakuum zur Trockne, nimmt den Rückstand in Methylenchlorid auf, wäscht zunächst mit Wasser und extrahiert anschließend die Titelverbindung mit zweimal 25 ml 2 N HCl. Die wäßrige Phase wird abgetrennt, mit Natronlauge alkalisch gestellt und die organische Base mit Methylenchlorid ausgeschüttelt. Die Methylenchloridphase wird eingedampft und der Rückstand im Vakuum destilliert.

Ausbeute : 10 g (61 % d.Th.) vom $Kp_{0,05}$ = 155-158 °C.

## Beispiel 6

(—)-1-Benzyl-4-diäthylaminomethyl-pyrrolidin-2-on

11,5 g (0,056 Mol) (—)-1-Benzyl-4-aminomethyl-pyrrolidin-2-on, 130 ml Wasser, 13 g Acetaldehyd, 5,8 ml konzentrierte Salzsäure und 6,5 g Pd/C 20 % werden 5 1/4 Stunden bei 5 bar und 25 °C hydriert. Der Rückstand wird eingedampft, in 30 ml Wasser aufgenommen und mit Methylenchlorid ausgeschüttelt. Die salzsaure, wäßrige Lösung wird alkalisch gestellt und ebenfalls mit Methylenchlorid extrahiert.

Durch Destillation im Kugelrohr erhält man

11,2 g (76,4 % d.Th.) der Titelverbindung

$\alpha_D$ = — 9,4 (c = 1,0 ; Methanol).

Analog Beispiel 6 erhält man durch Hydrierung von 8,4 g (0,041 Mol) (+)-1-Benzyl-4-aminomethyl-pyrrolidin-2-on, 95 ml Wasser, 9,5 Acetaldehyd, 4,2 ml konzentrierte Salzsäure und 4,7 g Pd/C 20 % das (+)-1-Benzyl-4-diäthylaminomethyl-pyrrolidin-2-on, $\alpha_D$ = + 9,4 (c = 1,0 ; Methanol).

Beispiel 7

1-(4-Fluor-benzyl)-4-N-methylpiperazinylmethylpyrrolidin-2-on

a) 24 g (0,11 Mol) 1-(4-Fluor-benzyl)-4-hydroxymethylpyrrolidin-2-on werden mit 10 ml (0,14 Mol) Thionylchlorid in 200 ml Methylenchlorid zunächst 10 Stunden und nach Zugabe von weiteren 10 ml Thionylchlorid weitere 6 Stunden unter Rückfluß gekocht. Unter Eiskühlung wird mit Ammoniak neutralisiert und nach dem Abtrennen der organischen Phase diese getrocknet und eingedampft. Es hinterbleiben 23 g (92 % d. Th.) eines rotbraunen Öls, das ohne weitere Reinigung verwendet wird.

b) 5 g (0,002 Mol) des obigen Öls werden mit 4,4 g (0,04 Mol) 1-Methyl-piperazin in 30 ml Dimethylformamid 1-2 Stunden unter Rückfluß gekocht. Man destilliert anschließend das Dimethylformamid weitgehend im Vakuum ab, nimmt den Rückstand in Methylenchlorid auf, wäscht mit Wasser, trocknet die organische Phase und dampft erneut ein. Der Rückstand wird über $SiO_2$ mit Methylenchlorid/Methanol 95 :5 als Fließmittel chromatographiert. Die Hauptfraktion wird eingedampft und der Rückstand (5 g) in 30 ml Methanol gelöst. Zu dieser Lösung werden 2,8 g Fumarsäure gegeben. 5,2 g (48 % d. Th.) der Titelverbindung scheiden sich kristallin als Fumarat ab. Fp. 179-180 °C.

Beispiel 8

1-(4-Fluorbenzyl)-4-morpholinomethyl-pyrrolidin-2-on

a) 8,9 g (0,04 Mol) 1-(4-Fluorbenzyl)-4-hydroxymethylpyrrolidin-2-on in 100 ml absolutem Methylenchlorid und 4,8 g Pyridin werden mit 6,9 g (0,06 Mol) Methansulfonsäurechlorid versetzt. Man kocht 2,5 Stunden unter Rückfluß, kühlt ab und schüttelt mit verdünntem Ammoniak und Wasser. Die organische Phase wird getrocknet und eingedampft. Man erhält 11 g (93 % d. Th.) rohen Ester vom Fp. 84-86 °C.

b) 6,7 g (0,023 Mol) Ester und 2,6 g (0,03 Mol) Morpholin werden in 20 ml Dioxan 2 Stunden unter Rückfluß gekocht. Man dampft das Lösungsmittel im Vakuum ab, nimmt den Rückstand in Methylenchlorid auf und extrahiert mit 50 ml 2 n Salzsäure. Die wäßrigen Auszüge werden mit Ammoniak alkalisch gestellt und die ölige Base mit Methylenchlorid ausgeschüttelt. Die Methylenchloridphase wird getrocknet und eingedampft. Den Rückstand (4,2 g) nimmt man in 30 ml Methanol auf und fügt in der Wärme 1,2 g Fumarsäure hinzu. Nach dem Abkühlen fällt das Fumarat der Titelverbindung kristallin aus.

Ausbeute : 7 g = 57 % d. Th. farblose Kristalle vom Fp. 175-176 °C.

Beispiel 9

1-(4-Fluorbenzyl)-4-aminomethyl-pyrrolidin-2-on

a) 4,0 g (0,013 Mol) Mesyl-Ester, hergestellt nach Beispiel 7, werden mit 2,8 g (0,015 Mol) Phthalimidkalium in 50 ml Dimethylformamid 30 Minuten unter Rückfluß gekocht. Man dampft im Vakuum ein, nimmt den Rückstand in Methylenchlorid auf, wäscht mit Wasser, trocknet die organische Phase und dampft erneut ein. Der Rückstand wird mit Äther verrieben und ergibt 3,6 g (78 % d. Th.) hellgraue Kristalle vom Fp. 124-125 °C.

b) 3,5 g (0,1 Mol) obiger Phthalimidverbindung werden zusammen mit 5,5 g Hydrazinhydrat in 200 ml Alkohol 4 Stunden bei Raumtemperatur gerührt. Die Aufarbeitung erfolgt wie in Beispiel 1 beschrieben. Es werden 2,5 g (89 % d. Th.) Fumarat der Titelverbindung vom Fp. 214-215 °C erhalten.

Die Titelverbindung kann auch erhalten werden, indem man 5 g (16 mMol) Mesyl-Ester (siehe Beispiel 7) in 100 ml Dimethylformamid löst und nach Zugabe von 1,3 g Natriumazid 2 Stunden auf 100 °C erwärmt, das nach entsprechender Aufarbeitung erhaltene Öl in Methanol mit Raney-Nickel hydriert und die Base wie oben in das Fumarat umwandelt. Ausbeute : 4,2 g (90 % d. Th.).

Analog der in den vorstehenden Beispielen beschriebenen Arbeitsweise wurden ferner die folgenden Endprodukte erhalten :

(Siehe Tabellen Seite 9 ff.)

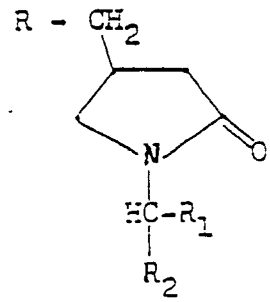

| Beispiel Nr. | R | $R_1$ | $R_2$ | Fp. °C / Kp. °C |
|---|---|---|---|---|
| 10 | $-NH_2$ | H | 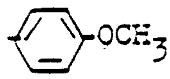 | Fp. 215 - 216 (Fumarat) |
| 11 | $-NH_2$ | H | 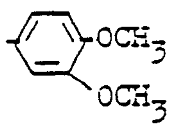 | Fp. 187 - 189 (Fumarat) |
| 12 | $-NH_2$ | H | 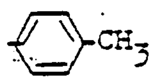 | Fp. 225 - 226 (Fumarat) |
| 13 | $-NH_2$ | H | 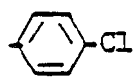 | Fp. 189 - 191 (Fumarat) |
| 14 | $-NH_2$ | H | 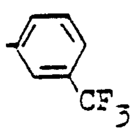 | Fp. 168 - 169 (Fumarat) |
| 15 | $-NH_2$ | H | 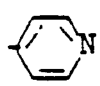 | Fp. 179 - 181 (Fumarat) |
| 16 | $-NH_2$ | $-CH_3$ | 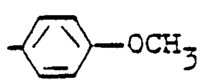 | Fp. 167 - 168 (Fumarat) |
| 17 | 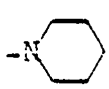 | H | 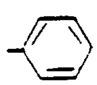 | Fp. 58 - 60<br>$Kp_{0,05}$ 180<br>(Base) |

| Beispiel Nr. | R | $R_1$ | $R_2$ | Fp. °C / Kp. °C |
|---|---|---|---|---|
| 18 | 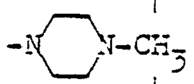 | H | 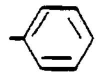 | Fp. 190 - 192 (Fumarat) |
| 19 | 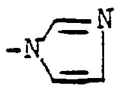 | H | 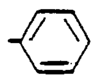 | $Kp._{0,05}$ 230 (Base) |
| 20 | $-HN-CH_3$ | H | 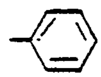 | $Kp._{0,05}$ 180 (Base) |
| 21 | $-NH_2$ | H | 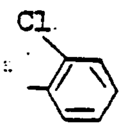 | Fp. 179 - 180 (Fumarat) |
| 22 | 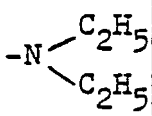 | H | 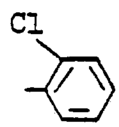 | $Kp._{0,05}$ 156 (Base) |
| 23 | 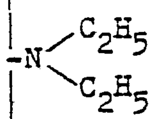 | H | 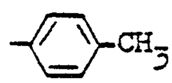 | $Kp._{0,05}$ 175 (Base) |

Beispiel 24

1-Benzyl-5-dimethylaminomethyl-pyrrolidin-2-on

a) Eine Lösung von 10,26 g (0,05 Mol) 1-Benzyl-5-hydroxymethyl-pyrrolidin-2-on (Fp. 76-77 °C) und 5,6 g (0,055 Mol) Triäthylamin in 80 ml Methylenchlorid wird mit 6,3 g (0,055 Mol) Methansulfonsäurechlorid in 20 ml Methylenchlorid versetzt. Das Reaktionsgemisch wird anschließend 1 Stunde unter Rückfluß erhitzt und nach dem Abkühlen mit Wasser ausgeschüttelt. Die organische Phase wird nach dem Trocknen über wasserfreiem Natriumsulfat am Rotationsverdampfer eingedampft. Man erhält 14,1 g (gelbes Öl) rohen 1-Benzyl-5-hydroxymethyl-pyrrolidin-2-on-methansulfonsäureester, der ohne weitere Reinigung in die nächste Reaktionsstufe eingesetzt wird.

b) 8,5 g (0,03 Mol) des nach a) erhaltenen Mesylats werden mit einer Lösung von 10 g Dimethylamin in 60 ml Dioxan in einem Autoklaven 3 Stunden auf 150 °C erhitzt. Die abgekühlte Reaktionsmischung wird im Vakuum zur Trockne eingeengt. Man löst den Rückstand in 2 n Salzsäure und extrahiert mit Äther. Die saure wäßrige Phase wird mit konzentriertem Ammoniak alkalisch gestellt und mit Methylenchlorid ausgeschüttelt. Die Methylenchloridlösung wird getrocknet und eingedampft. Der Rückstand (6,5 g) wird mit einer äquivalenten Menge Fumarsäure in das saure Fumarat der Titelverbindung überführt. Ausbeute : 6,4 g (61 % d. Th.) ; Fp. 137-138 °C.

(Siehe Tabelle Seite 11 ff.)

Analog Beispiel 25 wurden ferner hergestellt :

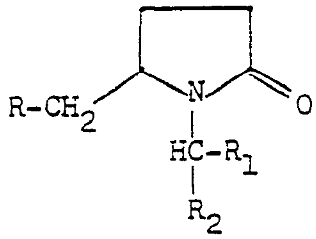

| Beispiel Nr. | R | $R_1$ | $R_2$ | Fp.°C / Kp.°C |
|---|---|---|---|---|
| 25 | $-N(C_2H_5)_2$ | H | 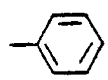 | Fp. 163 - 164 (Hydrochlorid) |
| 26 | 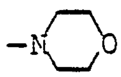 | H | 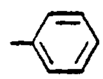 | Fp. 167 - 169 (Oxalat) |
| 27 | 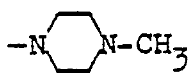 | H | 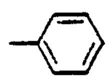 | Fp. 258 (Dihydrochlorid) |
| 28 | 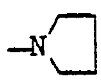 | H | 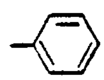 | Fp. 188 - 190 (Hydrochlorid) |
| 29 | $-N(CH_3)_2$ | H | 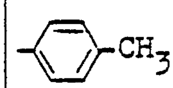 | Fp. 163 - 164 (Fumarat) |
| 30 | $-N(C_2H_5)_2$ | H | 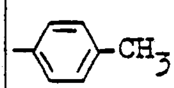 | Fp. 152 - 153 (Hydrochlorid) |
| 31 | $-N(CH_3)_2$ | H | 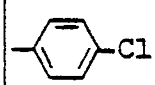 | Fp. 157 - 158 (Fumarat) |
| 32 | $-N(C_2H_5)_2$ | H | 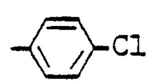 | Fp. 149 - 151 (Hydrochlorid) |

| Beispiel Nr. | R | $R_1$ | $R_2$ | Fp.°C / Kp.°C |
|---|---|---|---|---|
| 33 | $-N(CH_3)_2$ | H | 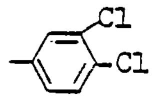 | Fp. 167 - 168 (Fumarat) |
| 34 | $-N(C_2H_5)_2$ | H | 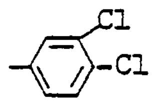 | Fp. 159 - 161 (Hydrochlorid) |
| 35 | $-N(CH_3)_2$ | H | 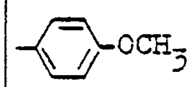 | Öl (Base) |
| 36 | $-N(C_2H_5)_2$ | H | 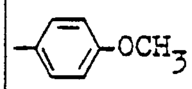 | Öl (Base) |

Beispiel 37

1-Benzyl-5-aminomethyl-pyrrolidin-2-on

16,4 g (0,07 Mol) 1-Benzyl-5-hydroxymethyl-pyrrolidin-2-on-methansulfonsäureester (siehe Beispiel 25 a)) werden in 200 ml Dimethylformamid gelöst und nach Zugabe von 4,6 g (0,07 Mol) Natriumazid 90 Minuten bei 100 °C gerührt. Nach Eindampfen, Verteilen zwischen Wasser und Methylenchlorid und Aufarbeiten der organischen Phase erhält man 13,8 g (92 % d.Th.) Öl, das roh weiter umgesetzt werden kann. Es wird in 200 ml Methanol gelöst und nach Zugabe von Raney-Nickel bei 20 °C und 5 bar hydriert. Nach Absaugen des Katalysators und Eindampfen des Filtrats werden 11 g (85 % d.Th.) Öl erhalten, die gelöst in Methanol und nach Zugabe von Fumarsäure das gewünschte Hemifumarat der Titelverbindung ergeben (Fp. 187-188 °C).

Pharmazeutische Formulierungsbeispiele

| A) Tabletten | pro Tablette |
|---|---|
| Wirkstoff | 100 mg |
| Milchzucker (pulverisiert) | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxy-methylstärke und das Magnesiumstearat, vermischt und verpreßt das Gemisch zu Tabletten geeigneter Größe.

C) Ampullen

| | | |
|---|---|---|
| 4-Aminomethyl-1-(4-fluorbenzyl)-pyrrolidin-2-on-Fumarat | | 50,0 mg |
| Natriumchlorid | | 10,0 mg |
| bidestilliertes Wasser | q.s. ad | 1,0 ml |

Herstellung

Der Wirkstoff und das Natriumchlorid werden in bidestilliertem Wasser gelöst und die Lösung in Ampullen steril abgefüllt.

D) Tropfen

| | | |
|---|---|---|
| 4-Aminomethyl-1-(4-fluorbenzyl)-pyrrolidin-2-on-Fumarat | | 5,0 g |
| p-Hydroxybenzoesäuremethylester | | 0,1 g |
| p-Hydroxybenzoesäurepropylester | | 0,1 g |
| entmineralisiertes Wasser | q.s. ad | 100,0 ml |

Herstellung

Der Wirkstoff und die Konservierungsmittel werden in demineralisiertem Wasser gelöst und die Lösung filtriert und in Flaschen zu je 100 ml abgefüllt.

**Patentansprüche**

1. 1-Benzyl-aminoalkyl-pyrrolidinone der allgemeinen Formel

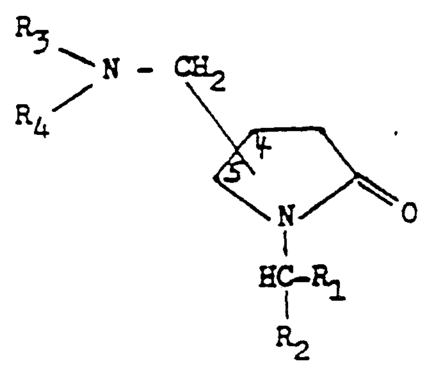

worin

$R_1$ Wasserstoff oder Methyl,

$R_2$ einen Phenylrest, der ein- oder zweifach durch Alkoxy mit 1 bis 2 Kohlenstoffatomen, verzweigtes oder unverzweigtes Alkyl mit 1 bis 4 Kohlenstoffatomen, Fluor, Chlor, Brom, Trifluormethyl, Hydroxy oder Nitro substituiert sein kann oder einen Pyridylrest ;

$R_3$ und $R_4$, die gleich oder verschieden sein können, Wasserstoff oder einen Alkylrest mit 1 bis 2 Kohlenstoffatomen,

$R_3$ und $R_4$, zusammen mit dem Stickstoffatom einen gesättigten 5- oder 6-Ring, der als weiteres Heteroatom ein O- oder N-Atom enthalten und gegebenenfalls durch Methyl substituiert sein kann oder einen Imidazolring bilden und die Aminoalkylgruppe in 4- oder 5-Stellung steht und deren Säureadditionssalze,

mit der Maßgabe, daß im Fall der Base wenn $R_1$ = Methyl, $R_2$ = Phenyl und die Aminomethylgruppe in 5-Stellung steht, $R_3$ und $R_4$ nicht beide Wasserstoff bedeuten können.

2. 1-Benzyl-4-aminoalkyl-pyrrolidinone der allgemeinen Formel I, worin $R_1$ Wasserstoff, $R_2$ einen gegebenenfalls durch Fluor oder Methoxy in p-Stellung substituierten Phenylrest und $R_3$ und $R_4$ Wasserstoff oder Methyl bedeuten und deren Säureadditionssalze.

3. 4-Aminomethyl-1-benzyl-pyrrolidin-2-on und dessen Säureadditionssalze.

4. 4-Aminomethyl-1-(p-fluorbenzyl)-pyrrolidin-2-on und dessen Säureadditionssalze.

5. 4-Aminomethyl-1-(p-methoxybenzyl)-pyrrolidin-2-on und dessen Säureadditionssalze.

6. 4-Diäthylaminomethyl-1-benzyl-pyrrolidin-2-on und dessen Säureadditionssalze.

7. 5-Diäthylaminomethyl-1-benzyl-pyrrolidin-2-on und dessen Säureadditionssalze.

8. 5-Aminomethyl-1-benzyl-pyrrolidin-2-on und dessen Säureadditionssalze.
9. Verfahren zur Herstellung der Verbindungen der allgemeinen Formel I

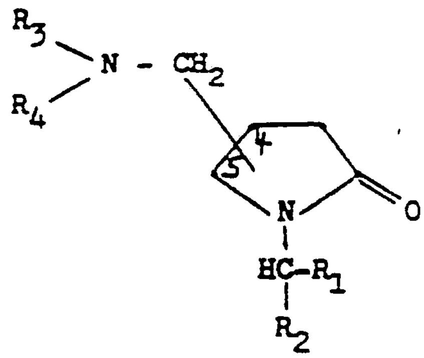

(I)

worin

$R_1$ Wasserstoff oder Methyl

$R_2$ einen Phenylrest, der ein- oder zweifach durch Alkoxy mit 1 bis 2 Kohlenstoffatomen, Fluor, Chlor, Brom, Trifluormethyl, verzweigtes oder unverzweigtes Alkyl mit 1 bis 4 Kohlenstoffatomen Hydroxy oder Nitro substituiert sein kann oder einen Pyridylrest und

$R_3$ und $R_4$, die gleich oder verschieden sein können, Wasserstoff, jedoch nicht beide Wasserstoff oder einen Alkylrest mit 1 bis 2 Kohlenstoffatomen bedeuten oder beide Reste, $R_3$ und $R_4$, zusammen mit dem Stickstoffatom einen gesättigten 5- oder 6-Ring, der als weiteres Heteroatom ein O- oder N-Atom enthalten und gegebenenfalls durch Methyl substituiert sein kann oder einen Imidazolring bilden und die Aminoalkylgruppe in 4- oder 5-Stellung steht,

dadurch gekennzeichnet, daß man ein Hydroxymethylpyrrolidin-2-on der allgemeinen Formel

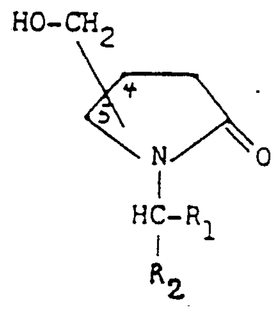

(II)

worin

$R_1$ und $R_2$ die oben angegebene Bedeutung haben, mit einem Thionyl- oder einem Phosphor-Halogenid oder einem Tosyl- oder Mesylhalogenid umsetzt und die so erhaltene Verbindung der allgemeinen Formel III

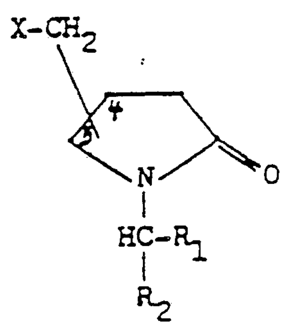

(III)

worin

$R_1$ und $R_2$ die oben angegebene Bedeutung haben und X ein Halogenatom oder den Tosyloxybeziehungsweise Mesyloxyrest bedeutet mit einem primären oder sekundären Amin der allgemeinen Formel

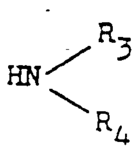

(IV)

worin

$R_3$ und $R_4$ die oben angegebene Bedeutung haben, umsetzt, und daß man gegebenenfalls ein so erhaltenes Endprodukt der allgemeinen Formel I nach üblichen Verfahren in ein physiologisch unbedenkliches Säureadditionssalz überführt.

10. Verfahren zur Herstellung neuer Verbindungen der allgemeinen Formel I

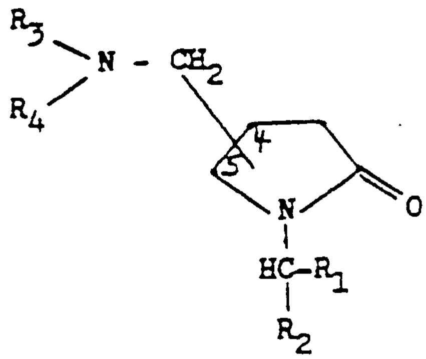

(I)

worin

$R_1$ Wasserstoff oder Methyl

$R_2$ einen Phenylrest, der ein- oder zweifach durch Alkoxy mit 1 bis 2 Kohlenstoffatomen, Fluor, Chlor, Brom, Trifluormethyl, verzweigtes oder unverzweigtes Alkyl mit 1 bis 4 Kohlenstoffatomen, Hydroxy oder Nitro substituiert sein kann oder einen Pyridylrest und

$R_3$ und $R_4$ Wasserstoff bedeuten,

dadurch gekennzeichnet, daß man

α) eine Verbindung der allgemeinen Formel III

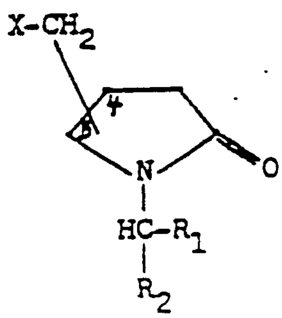

(III)

mit Phtalimid oder einem Alkalisalz des Phtalimids umsetzt und die so erhaltene 4-Phthalimidomethylverbindung mit Hydrazin umsetzt, oder

β) eine Verbindung der allgemeinen Formel V

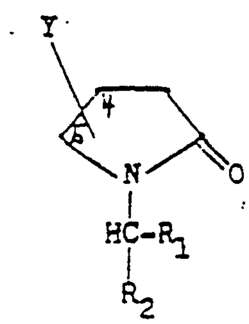

(V)

worin

$R_1$ und $R_2$ die oben angegeben Bedeutung haben und Y einen der Reste $—CH_2N_3$ oder $—CN$ bedeutet, hydriert und daß man gegebenenfalls ein so erhaltenes Endprodukt der allgemeinen Formel I nach üblichen Verfahren in ein physiologisch unbedenkliches Säureadditionssalz überführt.

11. Pharmazeutische Zubereitungen, enthaltend als Wirkstoff eine oder mehrere Verbindungen der allgemeinen Formel I oder deren physiologisch verträgliche Säureadditionssalze in Kombination mit üblichen Hilfs- und/oder Trägerstoffen.

12. Verfahren zur Herstellung von pharmazeutischen Präparaten gemäß Anspruch 11, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

13. Verwendung von 1-Benzyl-aminoalkylpyrrolidinonen der allgemeinen Formel

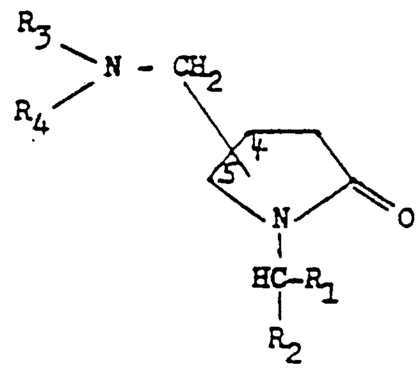

(I)

worin

$R_1$ Wasserstoff oder Methyl

$R_2$ einen Phenylrest, der ein- oder zweifach durch Alkoxy mit 1 bis 2 Kohlenstoffatomen, Fluor, Chlor, Brom, Trifluormethyl, verzweigtes oder unverzweigtes Alkyl mit 1 bis 4 Kohlenstoffatomen, Hydroxy oder Nitro substituiert sein kann oder einen Pyridylrest und

$R_3$ und $R_4$, die gleich oder verschieden sein können, Wasserstoff oder einen Alkylrest mit 1 bis 2 Kohlenstoffatomen bedeuten oder beide Reste, $R_3$ und $R_4$, zusammen mit dem Stickstoffatom einen gesättigten 5- oder 6- Ring, der als weiteres Heteroatom ein O- oder N-Atom enthalten und gegebenenfalls durch Methyl substituiert sein kann oder einen Imidazolring bilden und die Aminoalkylgruppe in 4- oder 5-Stellung steht und deren Säureadditionssalze, zur Herstellung eines Arzneimittels zur Behandlung von Zustandsformen eingeschränkter cerebraler Leistungsfähigkeit.

**Claims**

1. 1-benzyl-aminoalkyl-pyrrolidinones of general formula

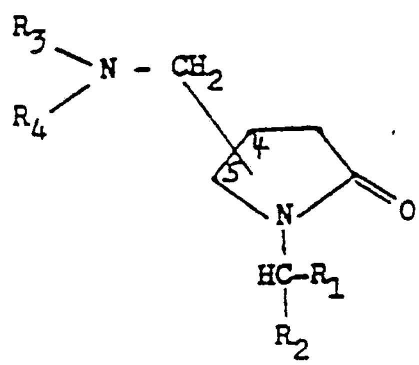

wherein

$R_1$ represents hydrogen or methyl,

$R_2$ represents a phenyl group which may be mono- or disubstituted by alkoxy with 1 to 2 carbon atoms, branched or unbranched alkyl with 1 to 4 carbon atoms, fluorine, chlorine, bromine, trifluoromethyl, hydroxy or nitro, or a pyridyl group and

$R_3$ and $R_4$, which may be identical or different, represent hydrogen or an alkyl group with 1 to 2 carbon atoms,

$R_3$ and $R_4$ together with the nitrogen atom form a saturated 5- or 6-membered ring which may contain an O or N atom as a further heteroatom and may optionally be substituted by methyl, or they form an imidazole ring and the aminoalkyl group is in the 4 or 5 position, and the acid addition salts thereof, subject to the proviso that in the case of the base, when $R_1$ represents methyl, $R_2$ represents phenyl and the aminomethyl group is in the 5 position, $R_3$ and $R_4$ cannot both represent hydrogen.

2. 1-Benzyl-4-aminoalkyl-pyrrolidinones of general formula I wherein $R_1$ represents hydrogen, $R_2$ represents a phenyl group optionally substituted by fluorine or methoxy in the p position and $R_3$ and $R_4$ represent hydrogen or methyl, and the acid addition salts thereof.

3. 4-Aminomethyl-1-benzyl-pyrrolidin-2-one and the acid addition salts thereof.
4. 4-Aminomethyl-1-(p-fluorobenzyl)-pyrrolidin-2-one and the acid addition salts thereof.
5. 4-Aminomethyl-1-(p-methoxybenzyl)-pyrrolidin-2-one and the acid addition salts thereof.
6. 4-Diethylaminomethyl-1-benzyl-pyrrolidin-2-one and the acid addition salts thereof.
7. 5-Diethylaminomethyl-1-benzyl-pyrrolidin-2-one and the acid addition salts thereof.
8. 5-Aminomethyl-1-benzyl-pyrrolidin-2-one and the acid addition salts thereof.
9. Process for preparing compounds of general formula I

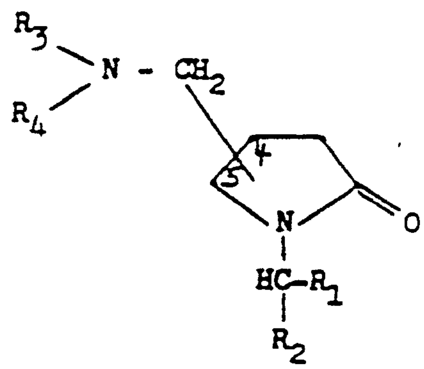

(I)

wherein

$R_1$ represents hydrogen or methyl,

$R_2$ represents a phenyl group which may be mono- or disubstituted by alkoxy with 1 to 2 carbon atoms, fluorine, chlorine, bromine, trifluoromethyl, branched or unbranched alkyl with 1 to 4 carbon atoms, hydroxy or nitro or a pyridyl group and

$R_3$ and $R_4$, which may be identical or different, represent hydrogen, however, they do not both represent hydrogen or an alkyl group with 1 to 2 carbon atoms or both groups, $R_3$ and $R_4$, together with the nitrogen atom form a saturated 5- or 6-membered ring which may contain an O or N atom as a further heteroatom and may optionally be substituted by methyl, or they form an imidazole ring and the aminoalkyl group is in the 4 or 5 position,

characterised in that a hydroxymethylpyrrolidin-2-one of general formula

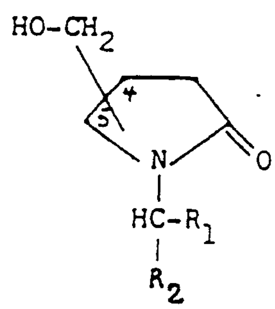

(II)

wherein

$R_1$ and $R_2$ are as hereinbefore defined is reacted with a thionyl or a phosphorus halide or a tosyl or mesyl halide and the resulting compound of general formula III

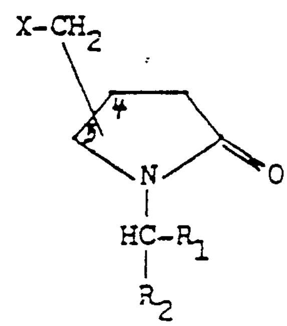

(III)

wherein

$R_1$ and $R_2$ are as hereinbefore defined and X represents a halogen atom or the tosyloxy or mesyloxy group, are reacted with a primary or secondary amine of general formula

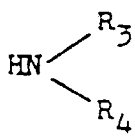

(IV)

wherein

$R_3$ and $R_4$ are as hereinbefore defined, and if desired an end product of general formula I thus obtained is converted by conventional methods into a physiologically acceptable acid addition salt.

10. Process for preparing new compounds of general formula I

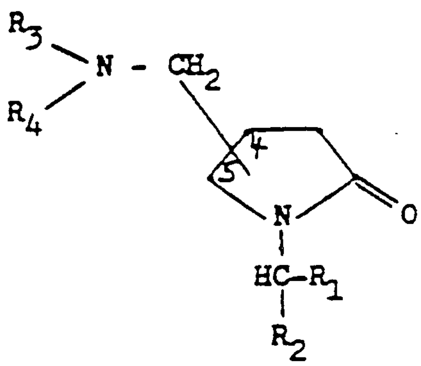

(I)

wherein

$R_1$ represents hydrogen or methyl,

$R_2$ represents a phenyl group which may be mono- or disubstituted by alkoxy with 1 to 2 carbon atoms, fluorine, chlorine, bromine, trifluoromethyl, branched or unbranched alkyl with 1 to 4 carbon atoms, hydroxy or nitro or a pyridyl group and

$R_3$ and $R_4$ represent hydrogen, characterised in that

α) a compound of general formula III

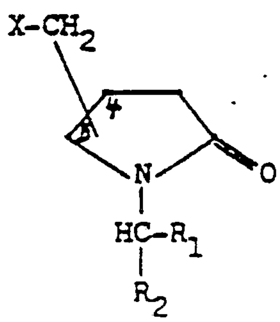

(III)

is reacted with phthalimide or an alkaline salt of phthalimide and the 4-phthalimidomethyl compound thus obtained is reacted with hydrazine or

β) a compound of general formula V

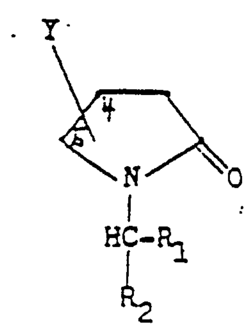

(V)

wherein

$R_1$ and $R_2$ are as hereinbefore defined and Y represents one of the groups $—CH_2N_3$ or $—CN$, is

hydrogenated, and if desired an end product of general formula I thus obtained is converted by conventional methods into a physiologically acceptable acid addition salt.

11. Pharmaceutical preparations containing as an active substance one or several compounds of general formula I or the physiologically tolerant acid addition salts thereof in conjunction with conventional excipients and/or carriers.

12. Process for producing pharmaceutical preparations as claimed in claim 11, characterised in that compounds of general formula I are processed with the usual galenic excipients and/or carriers to form conventional pharmaceutical preparations.

13. Use of 1-benzyl-aminoalkylpyrrolidinones of general formula

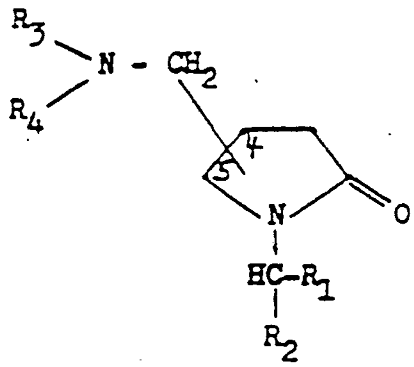

(I)

wherein

$R_1$ represents hydrogen or methyl,

$R_2$ represents a phenyl group which may be mono- or disubstituted by alkoxy with 1 to 2 carbon atoms, fluorine, chlorine, bromine, trifluoromethyl, branched or unbranched alkyl with 1 to 4 carbon atoms, hydroxy or nitro or a pyridyl group and

$R_3$ and $R_4$, which may be identical or different, represent hydrogen or an alkyl group with 1 to 2 carbon atoms or the two groups, $R_3$ and $R_4$, together with the nitrogen atom form a saturated 5- or 6-membered ring which may contain an O or N atom as a further heteroatom and if desired may be substituted by methyl or may form an imidazole ring and the aminoalkyl group is in the 4 or 5 position, and the acid addition salts thereof, for the preparation of a medicament for treating conditions of restricted cerebral performance.

**Revendications**

1. 1-benzyl-aminoalcoyl-pyrrolidinones de formule générale

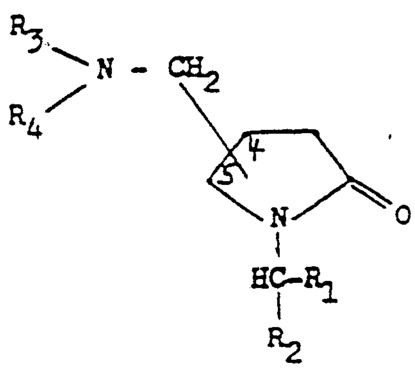

dans laquelle

$R_1$ remplace hydrogène ou méthyle.

$R_2$ remplace un radical phényle, qui peut être substitué une ou deux fois par alcoxy avec 1 à 2 atomes de carbone, alcoyle avec 1 à 4 atomes de carbone, ramifié ou non ramifié, fluor, chlore, brome, trifluorométhyle, hydroxy ou nitro ou remplace un radical pyridyle :

$R_3$ et $R_4$, qui peuvent être identiques ou différents, remplacent hydrogène ou un radical alcoyle avec 1 à 2 atomes de carbone.

$R_3$ et $R_4$, ensemble avec l'atome d'azote, forment un cycle saturé à 5 ou 6 chaînons qui peut contenir, en tant qu'autre hétéroatome, un atome de O ou de N et peut être éventuellement substitué par méthyle ou forment un cycle imidazole et le groupe aminoalcoyle se trouve en position 4 ou 5, et leurs sels d'addition d'acides,

étant entendu que, dans le cas de la base, lorsque $R_1$ = méthyle, $R_2$ = phényle et le groupe aminométhyle se trouve en position 5, $R_3$ et $R_4$ ne peuvent pas représenter l'un et l'autre hydrogène.

2. 1-benzyl-4-aminoalcoylpyrrolidinones de formule générale I, dans laquelle $R_1$ représente hydrogène, $R_2$ représente un radical phényle éventuellement substitué en position p par fluor ou méthoxy et $R_3$ et $R_4$ représentent hydrogène ou méthyle et leurs sels d'addition.

3. La 4-aminométhyl-1-benzyl-pyrrolidine-2-one et ses sels d'addition d'acides.

4. La 4-aminométhyl-1-(p-fluorobenzyl)-pyrrolidine-2-one et ses sels d'addition d'acides.

5. La 4-aminométhyl-1-(p-méthoxybenzyl)-pyrrolidine-2-one et ses sels d'addition d'acides.

6. La 4-diéthylaminométhyl-1-benzyl-pyrrolidine-2-one et ses sels d'addition d'acides.

7. La 5-diéthylaminométhyl-1-benzyl-pyrrolidine-2-one et ses sels d'addition d'acides.

8. La 5-aminométhyl-1-benzyl-pyrrolidine-2-one et ses sels d'addition d'acides.

9. Procédé pour la préparation des composés de formule générale I

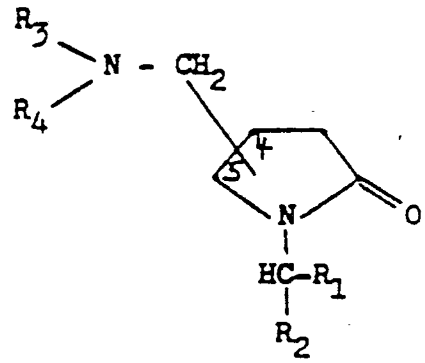

(I)

dans laquelle

$R_1$ remplace hydrogène ou méthyle,

$R_2$ remplace un radical phényle, qui peut être substitué une ou deux fois par alcoxy avec 1 à 2 atomes de carbone, fluor, chlore, brome, trifluorométhyle ou alcoyle avec 1 à 4 atomes de carbone, ramifié ou non ramifié, hydroxy ou nitro ou représente un radical pyridyle, et

$R_3$ et $R_4$, qui peuvent être identiques ou différents, représentent hydrogène, mais ne représentent toutefois pas l'un et l'autre hydrogène ou un radical alcoyle avec 1 à 2 atomes de carbone ou les deux radicaux $R_3$ et $R_4$, ensemble avec l'atome d'azote, forment un cycle saturé à 5 ou 6 chaînons qui contient en tant qu'autre hétéroatome, un atome de O ou de N et peut éventuellement être substitué par méthyle ou forment un cycle imidazole et le groupe aminoalcoyle est en position 4 ou 5,

caractérisé en ce que l'on fait réagir une hydroxyméthylpyrrolidine-2-one de formule générale II

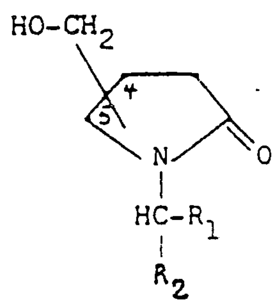

(II)

dans laquelle

$R_1$ et $R_2$ ont la signification indiquée plus haut, avec un halogénure de thionyle ou de phosphore ou un halogénure de tosyle ou de mésyle et on fait réagir le composé ainsi obtenu de formule générale III

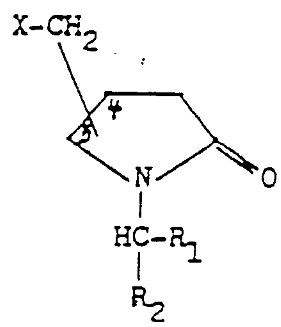

(III)

dans laquelle

$R_1$ et $R_2$ ont la signification indiquée plus haut et X représente un atome d'halogène ou le radical tosyloxy ou respectivement mésyloxy, avec une amine primaire ou secondaire de formule générale

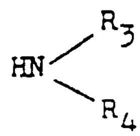

(IV)

dans laquelle

$R_3$ et $R_4$ ont la signification indiquée plus haut, et en ce qu'on transforme éventuellement un composé final ainsi obtenu de formule générale I selon des procédés usuels en un sel d'addition d'acide physiologiquement inoffensif.

10. Procédé pour la préparation des nouveaux composés de formule générale I

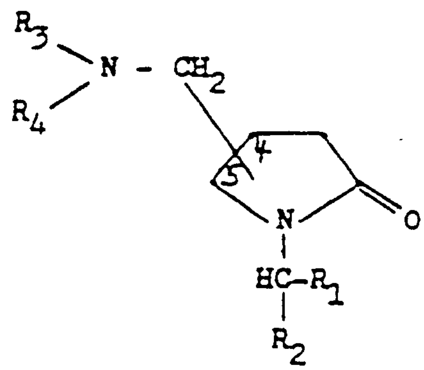

(I)

dans laquelle

$R_1$ représente hydrogène ou méthyle.

$R_2$ représente un radical phényle, qui peut être substitué une ou deux fois par alcoxy avec 1 à 2 atomes de carbone, fluor, chlore, brome, trifluorométhyle, alcoyle avec 1 à 4 atomes de carbone, ramifié ou non ramifié, hydroxy ou nitro ou représente un radical pyridyle, et

$R_3$ et $R_4$ représentent l'hydrogène, caractérisé en ce que

α) on fait réagir un composé de formule générale III

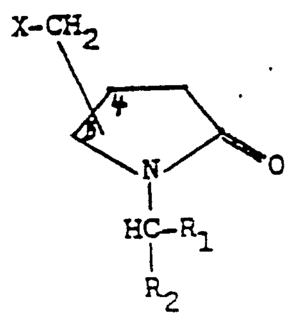

(III)

avec du phtalimide ou un sel alcalin du phtalimide et on fait réagir le composé 4-phtalimidométhylé ainsi obtenu avec de l'hydrazine, ou

β) on hydrogène un composé de formule générale V

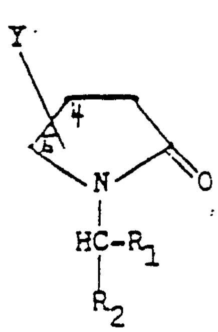

(V)

dans laquelle

$R_1$ et $R_2$ ont la signification indiquée plus haut et Y représente l'un des radicaux $—CH_2N_3$ ou $—CN$ et en ce qu'on transforme éventuellement un produit final ainsi obtenu de formule générale I, selon des procédés usuels, en un sel d'addition d'acide physiologiquement inoffensif.

11. Préparations pharmaceutiques contenant, en tant que substance active, un ou plusieurs composés de formule générale I ou leurs sels d'addition d'acides physiologiquement supportables en combinaison avec des adjuvants et/ou excipients usuels.

12. Procédé pour la fabrication de préparations pharmaceutiques selon la revendication 11, caractérisé en ce qu'on transforme des composés de formule générale I, au moyen d'adjuvants et/ou excipients galéniques usuels en formes d'utilisation pharmaceutiques usuelles.

13. Utilisation des 1-benzyl-aminoalcoylpyrrolidinones de formule générale

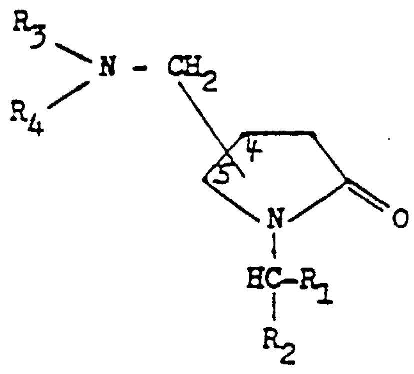

(I)

dans laquelle

$R_1$ représente hydrogène ou méthyle,

$R_2$ représente un radical phényle, qui peut être substitué une ou deux fois par alcoxy avec 1 à 2 atomes de carbone, fluor, chlore, brome, trifluorométhyle, alcoyle avec 1 à 4 atomes de carbone, ramifié ou non ramifié, hydroxy ou nitro ou remplace un radical pyridyle et

$R_3$ et $R_4$, qui peuvent être identiques ou différents, représentent hydrogène ou un radical alcoyle avec 1 à 2 atomes de carbone, ou les deux radicaux, $R_3$ et $R_4$, ensemble avec l'atome d'azote, forment un cycle saturé à 5 ou 6 chaînons, qui peut contenir en tant qu'autre hétéroatome, un atome de O ou de N et être éventuellement substitué par méthyle ou forment un cycle imidazole et le groupe aminoalcoyle est en position 4 ou 5, et de leurs sels d'addition d'acides, pour la fabrication d'un médicament pour le traitement des états de capacité cérébrale diminuée.